# EUROPEAN PATENT APPLICATION

(11) **EP 4 653 870 A1**
(43) Date of publication of application: **26.11.2025**
(21) Application number: 24744516.6
(22) Date of filing: 05.01.2024
(51) Int. Cl.: G01N 33/68, G01N 21/78, G01N 33/52

(54) **METHOD AND REAGENT FOR QUANTIFYING PROTEIN**

(30) Priority: 17.01.2023 JP 2023005239
(71) Applicant: Nitto Boseki Co., Ltd., Fukushima-shi, Fukushima 960-8161 (JP)
(72) Inventor: TOMARIGUCHI, Natsuki, Koriyama-shi, Fukushima 963-8061 (JP); WAKABAYASHI, Masayuki, Koriyama-shi, Fukushima 963-8061 (JP)
(74) Representative: Hoffmann Eitle
(86) International application number: PCT/JP2024/000038
(87) International publication number: WO 2024/154593

(57) **Abstract**

The present invention alleviates the problem of false high values in a method for quantifying protein by using a complex in which a metal is coordinated with a dye.

In this method for quantifying a protein in a specimen by using a complex in which a metal is coordinated with a dye, the protein is reacted with the complex in the presence of an aromatic carboxylic acid or a salt thereof.

## Description

### TECHNICAL FIELD

The present invention relates to a method and reagent for quantifying protein, and more specifically to a method and reagent for quantifying protein using a complex in which a metal is coordinated with a dye.

### BACKGROUND ART

In the field of clinical laboratory tests and the like, the concentration of total protein in a specimen is generally measured in order to obtain information on various diseases or symptoms, and the Kjeldahl method, the Lowry method, the Biuret method, the Coomassie Brilliant Blue method, and the like are known as methods of measuring protein. However, these methods are unsuitable for routine tests using automatic analysis, and they are affected by amino acids and thus unsuitable for measurement of proteins in urine, which contains a large amount of amino acids. Neither are they sufficiently sensitivity for measuring a trace amount of protein in urine or spinal fluid. For the quantitative analysis of protein in a specimen such as urine or spinal fluid, therefore, a method of measuring protein using a complex in which a metal is coordinated with a dye, such as a pyrogallol red-molybdenum complex, by utilizing a shift of an absorption wavelength of the complex in the presence of the protein has been used in many clinical laboratory chambers.

However, even in methods for quantifying protein using such a complex, some problems have been pointed out.

Tokuda et al. point out a problem that in methods using a complex composed of molybdenum and a dye such as pyrogallol red, oxalic acid, citric acid, phosphoric acid, or a salt thereof present in urine binds to molybdenum to cause negative errors, resulting in negative values in the urine of a healthy person (PATENT LITERATURE 1). As a means for solving this problem, Tokuda et al. propose adding a chelating agent that binds to molybdenum or a metal ion that does not react with a dye but can bind to oxalic acid, citric acid, phosphoric acid, or a salt thereof coexisting in a sample to reagents in advance (PATENT LITERATURE 1).

Tokuda et al. also point out that nitrite ions cause negative errors in methods for analyzing urinary protein, and propose adding specific nitrogen-containing organic compounds such as specific aniline derivatives or soluble salts thereof to assay reagents to solve that problem (PATENT LITERATURE 2).

Regarding the method of Tokuda et al. in which a chelating agent that binds to molybdenum or a metal ion capable of binding to chelating components coexisting in a sample is combined with a pyrogallol red-molybdenum complex, Kishi et al. point out the problems that the method requires long measurement time and is unsuitable for automatic analysis, and propose a method of reacting a pyrogallol red-molybdenum complex with protein in the presence of a polyhydric alcohol (PATENT LITERATURE 3).

These all report that certain components co-existing in urine react with a metal or dye forming a complex to prevent formation of the complex, thereby causing negative errors, particularly, negative values in a low range of protein concentration, and propose adding components that reduces the influence of the coexisting substances to an assay reagent.

### CITATION LIST

### PATENT LITERATURES

PATENT LITERATURE 1: JP-A-61-155757
PATENT LITERATURE 2: JP-A-63-235865
PATENT LITERATURE 3: JP-A-04-361160

### SUMMARY OF INVENTION

### TECHNICAL PROBLEM

In contrast, the present inventors have evaluated methods of measuring protein with a complex in which a metal is coordinated with a dye using patient specimens including those with abnormally high protein concentrations and found a new phenomenon of false high values in protein concentrations. Thus, an object of the present invention is to provide a method and reagent of improving the problem of false high values.

### SOLUTION TO PROBLEM

As a result of various studies on means for solving this problem, the present inventors have found that the problem of false high values can be improved by reacting protein with the complex in the presence of an aromatic carboxylic acid or a salt thereof. That is, the present invention provides the following methods and reagents.
[1] A method for quantifying protein in a specimen using a complex in which a metal is coordinated with a dye and of which an absorption wavelength shifts when the protein binds to the complex, the method comprising reacting the protein with the complex in the presence of an aromatic carboxylic acid or a salt thereof.
[2] A method for improving false high values when quantifying protein in a specimen using a complex in which a metal is coordinated with a dye and of which an absorption wavelength shifts when the protein binds to the complex, the method comprising reacting the protein with the complex in the presence of an aromatic carboxylic acid or a salt thereof.
[3] The method according to [1] or [2], wherein the aromatic carboxylic acid or the salt thereof is a monocyclic or bicyclic aromatic carboxylic acid or a salt thereof which has two or less carboxyl groups, and has no other substituents or an aromatic ring substituted with a sulfonic acid group, a hydroxyl group, a halogen, or an alkyl group having 1 to 4 carbon atoms.
[4] The method according to [1] or [2], wherein the aromatic carboxylic acid or the salt thereof comprises one or more selected from benzoic acid, hydroxybenzoic acid, aminobenzoic acid, salicylic acid, phthalic acid, and salts thereof.
[5] The method according to [4], wherein the aromatic carboxylic acid or the salt thereof comprises one or more selected from benzoic acid, hydroxybenzoic acid, salicylic acid, phthalic acid, and salts thereof.
[6] The method according to any one of [1] to [5], wherein the aromatic carboxylic acid or the salt thereof is contained in a reaction solution at a concentration of 4.2 mM or more.
[7] The method according to any one of [1] to [6], wherein the protein is reacted with the complex in the presence of:
   (1) an aliphatic amine having at least one substituent selected from an alkyl group having 1 to 4 carbon atoms, a hydroxylamino group having 1 to 4 carbon atoms and an aminoalkyl group having 1 to 4 carbon atoms; or a salt thereof (R-NH-R),
   (2) aniline, an aniline derivative having at least one substituent selected from a sulfonic acid group, a hydroxyl group, a sulfonylamino group, a halogen, an alkyl group having 1 to 4 carbon atoms, a hydroxylamino group having 1 to 4 carbon atoms and an amino group; or a salt thereof (R-NH-substituted or unsubstituted phenyl group or phenylene group-R),
   (3) hydrazine or a hydrazine derivative in which hydrazine is substituted with an alkyl group having 1 to 4 carbon atoms, or a salt thereof,
   (4) thiourea or a thiourea derivative in which thiourea is substituted with an alkyl group having 1 to 4 carbon atoms, or a salt thereof,
   (5) a polyhydric alcohol, and/or
   (6) a chelating agent,
   as well as the aromatic carboxylic acid or the salt thereof.
[8] The method according to any one of [1] to [7], wherein the dye is pyrogallol red or pyrocatechol violet, and the metal is molybdenum, tin or iron.
[9] The method according to any one of [1] to [8], wherein the specimen is urine or spinal fluid.
[10] A reagent for measuring protein, comprising: a complex in which a metal is coordinated with a dye and of which an absorption wavelength shifts when the protein binds to the complex; and an aromatic carboxylic acid or a salt thereof.
[11] The reagent for measuring protein according to [10], wherein the aromatic carboxylic acid or the salt thereof is a monocyclic or bicyclic aromatic carboxylic acid or a salt thereof which has two or less carboxyl groups, and has no other substituents or an aromatic ring substituted with a sulfonic acid group, a hydroxyl group, a halogen, or an alkyl group having 1 to 4 carbon atoms.
[12] The reagent for measuring protein according to [10], wherein the aromatic carboxylic acid or the salt thereof comprises one or more selected from benzoic acid, hydroxybenzoic acid, aminobenzoic acid, salicylic acid, phthalic acid, and salts thereof.
[13] The reagent for measuring protein according to [12], wherein the aromatic carboxylic acid comprises one or more selected from benzoic acid, hydroxybenzoic acid, salicylic acid, phthalic acid, and salts thereof.
[14] The reagent for measuring protein according to any one of [10] to [13], wherein the aromatic carboxylic acid or the salt thereof is contained at a concentration of 4.2 mM or more.
[15] The reagent for measuring protein according to any one of [10] to [14], wherein the dye is pyrogallol red or pyrocatechol violet, and the metal is molybdenum, tin or iron.
[16] The reagent for measuring protein according to any one of [10] to [15], further comprising:
   (1) an aliphatic amine having at least one substituent selected from an alkyl group having 1 to 4 carbon atoms, a hydroxylamino group having 1 to 4 carbon atoms, and an aminoalkyl group having 1 to 4 carbon atoms (R-NH-R); or a salt thereof;
   (2) aniline; an aniline derivative in which aniline is substituted with at least one substituent selected from a sulfonic acid group, a hydroxyl group, a sulfonylamino group, a halogen, an alkyl group having 1 to 4 carbon atoms, a hydroxylamino group having 1 to 4 carbon atoms, and an amino group (R-NH-substituted or unsubstituted phenyl or phenylene residue-R); or a salt thereof;
   (3) hydrazine; a hydrazine derivative in which hydrazine is substituted with an alkyl group having 1 to 4 carbon atoms (R-NH-NH₂); or a salt thereof;
   (4) thiourea; a thiourea derivative in which thiourea is substituted with an alkyl group having 1 to 4 carbon atoms(R-NH-C(=S)-N(-R)-R); or a salt thereof;
   (5) a polyhydric alcohol; and/or
   (6) a chelating agent.
[17] The reagent for measuring protein according to any one of [10] to [16], which is used for measuring protein in urine or spinal fluid.
[18] A method for producing a reagent for measuring protein, the method comprising dissolving a dye selected from pyrocatechol violet, pyrogallol red, bromopyrogallol red, o-hydroxyhydroquinonephthalein, and gallein; an oxyacid salt, a halide, a complex salt or an organic or inorganic acid salt of a metal selected from molybdenum, tin and iron; and an aromatic carboxylic acid or a salt thereof in a buffer solution.

### ADVANTAGEOUS EFFECTS OF INVENTION

With methods of measuring the concentration of total protein in a sample using a complex in which a metal is coordinated with a dye, such as a pyrogallol red-molybdenum complex, the problem of false high values is improved by performing the measurement in the presence of an aromatic carboxylic acid. This makes it possible to quantify the total protein more accurately, particularly in a high value range, and to improve the false-positive problem that may be a problem with conventional reagents.

### DESCRIPTION OF EMBODIMENTS

Embodiments of the present invention will be described in detail. However, the present invention should not be understood as being limited to the following embodiments.

A method for quantifying protein according to the present invention is characterized by reacting protein with a complex in which a metal is coordinated with a dye (hereinafter, abbreviated as "metal-dye complex" or "complex" in some cases) in the presence of an aromatic carboxylic acid or a salt thereof at the time of measuring protein in a sample using the complex. A reagent for measuring protein of the present invention is characterized by combining a metal-dye complex and an aromatic carboxylic acid in such a manner that both of them coexist at least in a reaction solution.

The dye constituting a metal-dye complex may be any dye which forms a complex through coordination with a metal and allows the absorption wavelength of the complex to shift due to protein binding thereto. Examples thereof include pyrocatechol violet, pyrogallol red, bromopyrogallol red, o-hydroxyhydroquinonephthalein, and gallein. The metal constituting a metal-dye complex may also be any metal which is coordinated with a dye to form a complex and allows the absorption wavelength of the complex to shift due to protein binding to the complex. Examples thereof include molybdenum, tin, iron, and aluminum. Specific examples of the metal-dye complex include a pyrocatechol violet-tin (IV) complex, a pyrogallol red-molybdenum complex, a bromopyrogallol red-molybdenum complex, an o-hydroxyhydroquinonephthalein-iron(III) complex, o-hydroxyhydroquinonephthalein-aluminum(III), and a gallein-molybdenum complex.

The concentration of a metal-dye complex in a reaction solution is set to be sufficient for the concentration of protein in the sample to be measured, and the concentration of a metal-dye complex in a reaction solution may be usually 0.030 to 0.090 mM, and preferably 0.037 to 0.057 mM. The concentration of a metal-dye complex in an assay reagent is designed to achieve such a metal-dye complex concentration in a reaction solution, and the concentration of a metal-dye complex in an assay reagent is usually adjusted to 0.030 to 0.090 mM, preferably 0.037 to 0.057 mM.

The reagent containing a metal-dye complex is prepared by dissolving such a dye and metal as described above in a buffer solution adjusted to a predetermined pH dependent on the type of a complex. The metal is mixed with the dye usually in the form of an oxyacid salt, a halide, a complex salt, or an organic or inorganic acid salt. Examples of such a form include molybdates (such as an alkali metal salt and an ammonium salt), stannates (such as an alkali metal salt and an ammonium salt), tin chloride, tin sulfate, ferrates (such as an alkali metal salt and ammonium salt), and iron chloride. In preparing the reagent, the amounts of a dye and a metal to be added to a buffer solution are determined according to the concentration of a complex to be formed, and the amount of a dye to be added may be usually 0.040 to 0.180 mM and preferably 0.054 to 0.081 mM in an assay reagent. The amount of a metal to be added may be usually 0.030 to 0.090 mM and preferably 0.037 to 0.057 mM in an assay reagent.

In a method according to the present invention, a metal-dye complex is brought into contact with protein in a sample in the presence of an aromatic carboxylic acid or a salt thereof to measure the protein, and the presence of the aromatic carboxylic acid improves the problem of false high values.

The aromatic carboxylic acid is an aromatic hydrocarbon having one or more carboxyl groups and preferably a monocyclic or bicyclic aromatic hydrocarbon having one or more carboxyl groups. The aromatic carboxylic acid is preferably an aromatic carboxylic acid having two or less carboxylic acids. The aromatic carboxylic acid may have a substituent other than the carboxylic acid; and examples thereof include an anionic functional group such as a sulfonic acid group, a hydroxyl group and a halogen; a cationic functional group such as a nitro group and an amino group; and an alkyl group (preferably an alkyl group having 1 to 4 carbon atoms). From the viewpoint of a large effect of improving false high values, the aromatic carboxylic acid used in the present invention is preferably an aromatic carboxylic acid having no substituent other than the carboxylic acid or substituted with an anionic functional group, and particularly preferably an aromatic carboxylic acid having no substituent other than the carboxylic acid.

Examples of the salt of an aromatic carboxylic acid include alkali metal salts such as a sodium salt and a potassium salt.

Specific examples of an aromatic carboxylic acid and a salt thereof used in the present invention include benzoic acid, methylbenzoic acid, hydroxybenzoic acid, 2-hydroxybenzoic acid, aminobenzoic acid, 4-aminobenzoic acid, salicylic acid, phthalic acid, isophthalic acid, terephthalic acid, mellitic acid, trimellitic acid, cinnamic acid, gallic acid, hybenzic acid, anthranilic acid, nicotinic acid, xylylic acid, mesitic acid, cumic acid, phenylacetic acid, atropic acid, hydrocinnamic acid, mesitylic acid, difenic acid, pyromellitic acid, hemellitic acid, durylic acid, α-isodylic acid, γ-isodylic acid, pranitic acid, cumic acid, ubitic acid, hemimellitic acid, trimesic acid, merophanic acid, anisic acid, asaronic acid, and salts thereof; and one or more of these can be combined. An aromatic carboxylic acid used in the present invention preferably includes one or more selected from benzoic acid, hydroxybenzoic acid, salicylic acid, phthalic acid, and salts thereof, more preferably includes one or more selected from benzoic acid, hydroxybenzoic acid, and salts thereof, and particularly preferably includes one or more selected from benzoic acid and a salt thereof.

The concentration of an aromatic carboxylic acid or a salt thereof in a reaction solution may be determined according to a measurement parameter to be applied so as to obtain a desired effect of improving false high values, and as demonstrated in EXAMPLES described later, the effect of improving false high values is increased depending on the concentration of an aromatic carboxylic acid. Therefore, according to a measurement parameter to be applied, the concentration may be appropriately set to a certain level or more, and the concentration of an aromatic carboxylic acid in a reaction solution may be usually 4.2 mM or more, preferably 7.0 mM or more, and more preferably 9.8 mM or more. The concentration of an aromatic carboxylic acid or a salt thereof in an assay reagent is designed to achieve such a desired concentration of aromatic carboxylic acid in a reaction solution, and is usually adjusted to a concentration of 4.2 mM or more, preferably 7.0 mM or more, and more preferably 9.8 mM or more.

On the other hand, the upper limits of the concentrations in a reaction solution and an assay reagent is preferably determined in consideration of the solubility of an aromatic carboxylic acid or a salt thereof to be used in the assay reagent, and the concentration of an aromatic carboxylic acid in a reaction solution is preferably 20.0 mM or less, more preferably 17.0 mM or less, and particularly preferably 15.0 mM or less. Similarly, the concentration of an aromatic carboxylic acid in an assay reagent is preferably 20.0 mM or less, more preferably 17.0 mM or less, and still more preferably 15.0 mM or less.

Therefore, the concentration of an aromatic carboxylic acid in a reaction solution is preferably 4.2 mM to 20.0 mM, more preferably 7.0 mM to 17.0 mM, and particularly preferably 9.8 mM to 15.0 mM. Similarly, the concentration of an aromatic carboxylic acid in an assay reagent is preferably 4.2 mM to 20.0 mM, more preferably 7.0 mM to 17.0 mM, and particularly preferably 9.8 mM to 15.0 mM.

An aromatic carboxylic acid or a salt thereof is only required to be present when a metal-dye complex react with protein, and, thus, it may be added to a reagent containing a metal-dye complex, to a specimen such as urine or a specimen diluted solution, or to a mixture of an assay reagent and a specimen at the time of mixing them. From the viewpoint of practical use, it is convenient to add an aromatic carboxylic acid or a salt thereof to a reagent containing a metal-dye complex in advance.

**In** a method and a reagent according to the present invention, reaction between a metal-dye complex and a protein in the presence of an aromatic carboxylic acid or a salt thereof can improve false high values particularly in a relatively high range of protein concentration, but in order to reduce negative errors at a low range of protein concentration, further, an aliphatic amine or a salt thereof, aniline, an aniline derivative or salts thereof, hydrazine, a hydrazine derivative or salts thereof, thiourea, a thiourea derivative or salts thereof, a polyhydric alcohol, and/or a chelating agent may be used together with the aromatic carboxylic acid.

Examples of the aliphatic amine include aliphatic amines having at least one substituent selected from an alkyl group having 1 to 4 carbon atoms, a hydroxylamino group having 1 to 4 carbon atoms, and an aminoalkyl group having 1 to 4 carbon atoms (R-NH-R, wherein two Rs are each independently a hydrogen atom or the above-described substituent, and at least one R is not a hydrogen atom); and examples of the salt thereof include a hydrochloride and a sulfate. Specific examples of the compound include N-methyl-N-ethanolamine, 2-amino-2-methyl-1-propanol, 1-aminopropanol, and hydrochlorides or sulfates thereof.

Examples of the aniline derivative include compounds in which aniline is substituted with at least one substituent selected from a sulfonic acid group, a hydroxyl group, a sulfonylamino group, halogen, an alkyl group having 1 to 4 carbon atoms, a nitro group, a hydroxylamino group having 1 to 4 carbon atoms, and an amino group (R-NH-substituted or unsubstituted phenyl or phenylene residue-R, wherein two Rs are each independently a hydrogen atom or the above-described substituent, and at least one R is not a hydrogen atom); preferred examples thereof include compounds in which NH₂ of aniline is unsubstituted or substituted with an alkyl group having 1 to 4 carbon atoms, a hydroxylamino group having 1 to 4 carbon atoms, or an amino group, and the phenyl group of aniline is unsubstituted or substituted with any one of a sulfonic acid group, a hydroxyl group, a sulfonylamino group, halogen, an alkyl group having 1 to 4 carbon atoms, and a nitro group; and examples of the salt thereof include a hydrochloride and a sulfate. Specific examples of the compound include sulfanilic acid, sulfanylamide, aniline-2,5-disulfonic acid, 2-aminophenol-4-sulfonic acid, 2-chloroaniline-4-sulfonic acid, 4-chloroaniline-3-sulfonic acid, 2,5-dichloroaniline-4-sulfonic acid, P-aminophenol, P-chloroaniline, hydroxyethylaniline, 3-hydroxy-4-nitroaniline, and hydrochlorides or sulfates thereof.

Examples of the hydrazine derivative include compounds in which hydrazine is substituted with an alkyl group having 1 to 4 carbon atoms (R-NH-NH₂, wherein R is an alkyl group having 1 to 4 carbon atoms), and examples of the salt thereof include a hydrochloride and a sulfate. Specific examples of the compound include phenylhydrazine and hydrazine hydrochloride.

Examples of the thiourea derivative include compounds in which thiourea is substituted with an alkyl group having 1 to 4 carbon atoms or a phenyl group (R-NH-C(=S)-N(-R)-R, wherein three Rs are each independently a hydrogen atom, an alkyl having 1 to 4 carbon atoms, or a phenyl group, and at least one R is not a hydrogen atom); and examples of the salt thereof include a hydrochloride and a sulfate. Specific examples of the compound include phenylthiourea, hydroxyethylaniline, 3-hydroxy-4-nitroaniline, and hydrochlorides or sulfates thereof.

The concentration of the aliphatic amine or the salts thereof, the aniline, the aniline derivative or the salts thereof, the hydrazine, and the hydrazine derivative or the salts thereof in a reaction solution may be determined according to assumed nitrite ion concentrations in a specimen, and the concentration in a reaction solution is usually 0.05 to 300 mM and preferably 0.24 to 60 mM. Therefore, the concentration of the aliphatic amine or the salt thereof, the aniline, the aniline derivative or the salts thereof, the hydrazine, and the hydrazine derivative or the salts thereof in an assay reagent is usually 0.05 to 300 mM and preferably 0.24 to 60 mM. The aliphatic amine or the salt thereof, the aniline, the aniline derivative, or the salts thereof, the hydrazine, and the hydrazine derivative or the salts thereof may also be only present at the time a metal-dye complex reacts with protein. Therefore, these compounds may be added to an assay reagent containing a metal-dye complex, to a specimen such as urine or a specimen-diluted solution, or to a mixture of an assay reagent and a specimen at the time of mixing them. From the viewpoint of practical use, it is convenient to add these compounds to an assay reagent containing a metal-dye complex in advance.

Examples of the polyhydric alcohol include glycols and sugar alcohols, and specific examples thereof include mannitol, sorbitol, dulcitol, glycerol, and polyglycerol. These polyhydric alcohols can be used singly or in combination of two or more thereof. The concentration of the polyhydric alcohol in a reaction solution may be determined according to the assumed content of a chelating component in a specimen, and the concentration of the same in a reaction solution is usually 2 to 100 mM and preferably 5 to 50 mM. Therefore, the concentration of the polyhydric alcohol in an assay reagent is usually 2 to 100 mM and preferably 5 to 50 mM. The polyhydric alcohol may also be only present at the time a metal-dye complex reacts with protein. Therefore, the polyhydric alcohol may be added to an assay reagent containing a metal-dye complex, to a specimen such as urine, or a mixture of an assay reagent and a specimen at the time of mixing them. From the viewpoint of practical use, it is convenient to add the polyhydric alcohol to an assay reagent containing a metal-dye complex in advance.

Examples of the chelating agent include ethylenediaminetetraacetic acid (EDTA), hydroxyethylethylenediaminetriacetic acid (EDTA-OH), ethylenediaminediacetic acid (EDDA), iminodiacetic acid (IDA), nitrilopropionic acid (NTP), nitrilotriacetic acid (NTA), hydroxyethyliminodiacetic acid (HIDA), citric acid, tartaric acid, oxalic acid, 1-hydroxyethane-1,1-diphosphonic acid, pyrophosphoric acid, hexametaphosphoric acid, tripolyphosphoric acid, metaphosphoric acid, and salts thereof. These chelating agents can be used singly or in combination of two or more thereof. Examples of the salt include alkali metal salts such as sodium, potassium, and lithium, and ammonium salts. The concentration of the chelating agent in a reaction solution and an assay reagent varies depending on the chelating agent to be used, but the concentration of the chelating agent in a reaction solution is usually 0.13 to 31 mM and preferably 0.25 to 2.5 mM. Similarly, the concentration of the chelating agent in an assay reagent is usually 0.13 to 31 mM and preferably 0.25 to 2.5 mM.

In addition to the above-described components, other components generally used in measuring protein may be contained. For example, various surfactants are used in order to prevent contamination of reaction tubes, assay cells and the like and to reduce the influence of hemolysis, chyle and the like, but a reagent according to the present invention may also contain various surfactants depending on their purposes. Surfactants are usually added to an assay reagent at a concentration of about 0.01 to 2 mass%.

An assay reagent according to the present invention can be prepared by dissolving the components described above in a buffer solution adjusted to a predetermined pH. The buffer solution is selected according to a metal-dye complex to be used, and examples thereof include a glycine buffer solution, a maleate buffer solution, a succinate buffer solution, a citrate buffer solution, an oxalate buffer solution, and a phosphate buffer solution. The pH of an assay reagent (a buffer solution) may be selected according to a metal-dye complex to be used in such a way that complex formation and absorption wavelength shift appropriately occur. For example, in the case of a pyrogallol-molybdenum complex, a pH of 2 to 2.5, preferably a pH of 2.2, is selected.

A method of the present invention can be performed in the same manner as a conventional method using a metal-dye complex, except that the reaction of a metal-dye complex with protein is performed in the presence of an aromatic carboxylic acid or a salt thereof. For example, an assay reagent containing the above-described components are mixed with a specimen in which the concentration of protein is to be quantified, and they are reacted at a constant temperature of room temperature to 37°C for a constant time (for example, about 3 to 15 minutes). The specimen to be used for quantifying the protein concentration may be diluted as necessary, so that the diluted sample may be subjected to the measurement. When protein binds to a metal-dye complex, the absorption wavelength of the complex shifts. Therefore, near a maximum absorption wavelength after the shift, a wavelength is selected as a main wavelength and, optionally, a secondary wavelength is selected, and the absorbance is then measured. On the other hand, a standard sample having a known protein concentration is used instead of specimens, and the absorbance is measured in the same manner to create a calibration curve. The protein concentration in a specimen can be determined by applying the absorbance obtained from the specimen to the calibration curve.

A method of the present invention can be used for quantification of protein in various specimens, and, because of its high sensitivity, particularly suitable for measurement of specimens having generally low protein concentrations, such as urine and spinal fluid. Measurement can be easily performed by colorimetric methodology, particularly in a short time, and is suitable for measurement using an automatic analyzer. The present invention can also be applied to the measurement of protein by a dipstick method.

### EXAMPLES

Hereinafter, the present invention will be more specifically described with reference to Examples; however, the present invention is not limited thereto.

### 1. Study on effect of improving false high values by addition of aromatic carboxylic acid

### 1-1. Preparation of reagent

### [Example 1]

To a 100 mM glycine buffer solution, 0.068 mM of pyrogallol red, 0.047 mM of ammonium molybdate, 12.65 mM of sodium benzoate, 13.47 mM of D-mannitol, 0.81 mM of sulfanilic acid, 0.04% EDTA-OH, and 0.01% surfactant were added and mixed, and the pH was adjusted to 2.2 to prepare an assay reagent containing a complex of pyrogallol red and molybdenum.

### [Examples 2 to 4]

Assay reagents were prepared in the same manner as in Example 1, except that potassium benzoate (Example 2), p-hydroxybenzoic acid (Example 3), and p-aminobenzoic acid (Example 4) were each added instead of 12.65 mM of sodium benzoate.

### [Comparative Example 1]

An assay reagent was prepared in the same manner as in Example 1, except that 12.65 mM of sodium benzoate was not added.

The compositions of the assay reagents of Examples 1 to 4 and Comparative Example 1 are summarized below.

**[Table 1]**

| | Example 1 | Example 2 | Example 3 | Example 4 | Comparative Example 1 |
|---|---|---|---|---|---|
| Glycine | 100 mM | 100 mM | 100 mM | 100 mM | 100 mM |
| Pyrogallol red | 0.068 mM | 0.068 mM | 0.068 mM | 0.068 mM | 0.068 mM |
| Ammonium molybdate | 0.047 mM | 0.047 mM | 0.047 mM | 0.047 mM | 0.047 mM |
| Sodium benzoate | 12.65 mM | - | - | - | - |
| Potassium benzoate | - | 12.65 mM | - | - | - |
| p-Hydroxybenzoic acid | - | - | 12.65 mM | - | - |
| p-Aminobenzoic acid | - | - | - | 12.65 mM | - |
| D-Mannitol | 13.47 mM | 13.47 mM | 13.47 mM | 13.47 mM | 13.47 mM |
| Sulfanilic acid | 0.81 mM | 0.81 mM | 0.81 mM | 0.81 mM | 0.81 mM |
| EDTA-OH | 0.04% | 0.04% | 0.04% | 0.04% | 0.04% |
| Surfactant | 0.01% | 0.01% | 0.01% | 0.01% | 0.01% |

### 1-2. Evaluation by spike recovery test

For the assay reagents of Examples 1 to 4 and Comparative Example 1, the effect of improving false high values by addition of an aromatic carboxylic acid was evaluated by a spike recovery test.

In this test, four urine specimens were used, a human serum albumin (HSA) solution having a known concentration was added thereto, the protein concentrations before and after the addition were measured using each reagent, and the recovery rate was determined from the measured values. The details are as follows.

### (1) Preparation of human serum albumin (HSA) stock solution

Human serum albumin (HSA) was weighed and dissolved in ion-exchanged water to prepare a 1500 mg/dl HSA solution. However, since HSA formulations usually contain about 20% moisture, the weighed value is not an accurate amount of HSA. Therefore, the HSA concentration of the prepared HSA stock solution was quantified by HPLC under the following conditions to confirm that the HSA concentration was accurately 1311 mg/dl.

### <Measurement conditions>

HPLC (Agilent OpenLAB CDS)
Eluent flow path pump: G7129A 1260 Vialsampler
Detector: G7114A 1260 VWD
Column: Yarratm 3 µm SEC-2000, LC Column 300 × 7.8 mm, Ea
Eluent: 100 mM PO4 buffer, pH 7.0
Column temperature: 35°C:
   Flow rate: 1.00 mL/min
   Standard: NIST 7% BSA

### (2) Preparation of test sample

Four urine specimens having different protein concentrations were used, and HSA stock solutions were added to these specimens in such amounts that the HSA concentrations achieved 100 mg/dl and 300 mg/dl based on the weighed value. The HSA concentrations based on the weighed value were then calculated to be 87.4 mg/dl and 262.2 mg/dl, respectively, from the HSA quantitative value in the HSA stock solution by the above-mentioned HPLC method.

### (3) Quantification of total protein

The total protein in four urine specimens and samples obtained by adding HSA stock solutions to these specimens in such amounts that the HSA concentrations achieved 87.4 mg/dl and 262.2 mg/dl was quantified using Hitachi type 7180 automatic analyzer (Hitachi, Ltd.). As a calibrator, an HSA solution quantified by HPLC was used, and the following measurement parameters were used.

| | |
|---|---|
| Sample volume: | 2.0 µL (Comparative Example and each Example) |
| First reagent volume: | 250 µL |
| Measurement wavelength (main)/(sub) | 600 nm/660 nm |
| Analytical method | one-point end method |
| Photometric point | 34 |

The measurement results are shown below.

**[Table 2]**

| | **Comparative Example 1** No aromatic carboxylic acid | **Example 1** Sodium benzoate |
|---|---|---|
| **Urine specimen 1** | | |
| No HAS addition | 142.2 | 135.1 |
| 87.4 mg/dl HAS addition | 236.6 | 218.1 |
| 262.2 mg/dl HAS addition | 449.8 | 384.9 |

| **Urine specimen 2** | | |
|---|---|---|
| No HAS addition | 223.0 | 191.2 |
| 87.4 mg/dl HAS addition | 318.2 | 277.7 |
| 262.2 mg/dl HAS addition | 528.1 | 434.1 |

**[Table 3]**

| | **Comparative Example 1** No aromatic carboxylic acid | **Example 2** Potassium benzoate | **Example** 3 p-Hydroxyben zoic acid | **Example 4** p-Aminobenzo ic acid |
|---|---|---|---|---|
| **Urine specimen 3** | | | | |
| No HAS addition | 142.7 | 134.3 | 135.5 | 134.4 |
| 87.4 mg/dl HAS addition | 248.3 | 221.8 | 223.6 | 222.7 |
| 262.2 mg/dl HAS addition | 474.7 | 382.0 | 386.9 | 386.1 |

| **Urine specimen 4** | | | | |
|---|---|---|---|---|
| No HAS addition | 223.5 | 190.4 | 192.7 | 198.0 |
| 87.4 mg/dl HAS addition | 337.5 | 273.9 | 279.2 | 282.1 |
| 262.2 mg/dl HAS addition | 561.6 | 431.2 | 435.6 | 440.7 |

### (4) Calculation of recovery rate

The recovery rate was calculated from the total protein concentration of each sample obtained by measurement using each reagent according to the following formula.
- Recovery rate of the samples to which 87.4 mg/dl of HSA was added [(Total protein concentration in sample to which 87.4 mg/dl of HSA was added - Total protein concentration in sample before addition of HSA)/87.4] × 100

- Recovery rate of samples to which 262.2 mg/dl of HSA was added [(Total protein concentration in sample to which 262.2 mg/dl of HSA was added - Total protein concentration in sample before addition of HSA)/262.2] × 100

The recovery rate of each sample calculated from the total protein concentration (mg/dL) of the urine specimen measured with each reagent is shown below.

**[Table 4]**

| | **Comparative Example 1** No aromatic carboxylic acid | **Example 1** Sodium benzoate |
|---|---|---|
| **Urine specimen 1** | | |
| Recovery rate in case of addition of 87.4 mg/dl HAS | 108% | 95% |
| Recovery rate in case of addition of 262.2 mg/dl HAS | 117% | 102% |

| **Urine specimen 2** | | |
|---|---|---|
| Recovery rate in case of addition of 87.4 mg/dl HAS | 109% | 99% |
| Recovery rate in case of addition of 262.2 mg/dl HAS | 116% | 93% |

**[Table 5]**

| | **Comparative Example 1** No aromatic carboxylic acid | **Example 2** Potassium benzoate | **Example 3** p-Hydroxyben zoic acid | **Example 4** p-Aminobenzo ic acid |
|---|---|---|---|---|
| **Urine specimen 3** | | | | |
| Recovery rate in case of addition of 87.4 mg/dl HAS | 121% | 100% | 101% | 101% |
| Recovery rate in case of addition of 262.2 mg/dl HAS | 127% | 94% | 96% | 96% |

| **Urine specimen 4** | | | | |
|---|---|---|---|---|
| Recovery rate in case of addition of 87.4 mg/dl HAS | 120% | 95% | 99% | 96% |
| Recovery rate in case of addition of 262.2 mg/dl HAS | 126% | 92% | 93% | 93% |

As described above, when measurement was performed with the reagent of Comparative Example 1, the recovery rate largely exceeded 100%, and the degree of deviation tended to increase as the protein concentration increased. On the other hand, when measurement was performed with the reagents of Examples 1 to 4, the recovery rate was around 100%, and a significant improvement was observed. From the above, it is understood that when an aromatic carboxylic acid is contained in the reagent, false high values are improved, so that the total protein can be quantified more accurately.

### 2. Study on effective concentration of aromatic carboxylic acid

### 2-1. Preparation of reagent

### [Examples 5 to 9]

Assay reagents were prepared in the same manner as in Example 1, except that sodium benzoate was added at a concentration of 2.8 mM, 5.6 mM, 8.4 mM, 11.2 mM, or 14.0 mM instead of 12.65 mM of sodium benzoate.

### 2-2. Measurement of total protein

Using the reagents of Examples 5 to 9 and Comparative Example 1, the total protein concentration (mg/dL) in two urine specimens (Specimens 3 and 4) and samples obtained by adding HSA stock solutions to these specimens in such amounts that the HSA concentrations achieved 87.4 mg/dl and 262.2 mg/dl was quantified in the same manner as in Example 1. The measurement results are summarized in the following table.

**[Table 6]**

| | **Comparative Example 1** Sodium benzoate 0 mM | **Example 5** Sodium benzoate 2.8 mM | **Example 6** Sodium benzoate 5.6 mM | **Example 7** Sodium benzoate 8.4 mM | **Example 8** Sodium benzoate 11.2 mM | **Example 9** Sodium benzoate 14.0 mM |
|---|---|---|---|---|---|---|
| **Urine specimen 3** | | | | | | |
| No HAS addition | 142.7 | 139.4 | 136.2 | 134.6 | 135.1 | 134.6 |
| 87.4 mg/dl HAS addition | 248.3 | 241.3 | 231.6 | 227.1 | 223.9 | 223.1 |
| 262.2 mg/dl HAS addition | 474.7 | 452.0 | 421.3 | 400.6 | 386.5 | 383.3 |

| **Urine specimen 4** | | | | | | |
|---|---|---|---|---|---|---|
| No HAS addition | 223.5 | 218.3 | 206.3 | 196.0 | 191.7 | 189.1 |
| 87.4 mg/dl HAS addition | 337.5 | 324.7 | 299.0 | 285.1 | 229.9 | 276.1 |
| 262.2 mg/dl HAS addition | 561.6 | 533.3 | 484.6 | 449.8 | 437.4 | 429.9 |

### 2-3. Calculation of recovery rate

The recovery rate was calculated from the total protein concentration of each sample obtained by measurement using each reagent according to the above-described formula. The calculated recovery rates of the samples are summarized in the following table.

**[Table 7]**

| | **Comparative Example 1** Sodium benzoate 0 mM | **Example 5** Sodium benzoate 2.8 mM | **Example 6** Sodium benzoate 5.6 mM | **Example 7** Sodium benzoate 8.4 mM | **Example 8** Sodium benzoate 11.2 mM | **Example 9** Sodium benzoate 14.0 mM |
|---|---|---|---|---|---|---|
| **Urine specimen 3** | | | | | | |
| Recovery rate at the time of addition of 87.4 mg/dl HAS | 121% | 117% | 109% | 106% | 102% | 101% |
| Recovery rate at the time of addition of 262.2 mg/dl HAS | 127% | 119% | 109% | 101% | 96% | 95% |

| **Urine specimen 4** | | | | | | |
|---|---|---|---|---|---|---|
| Recovery rate at the time of addition of 87.4 mg/dl HAS | 120% | 122% | 106% | 102% | 101% | 100% |
| Recovery rate at the time of addition of 262.2 mg/dl HAS | 126% | 120% | 106% | 97% | 94% | 92% |

As described above, as compared with measurement with the reagent of Comparative Example 1, in which the concentration of sodium benzoate was 0 mM, the measurement with the reagent of Example 5, containing 2.8 mM of sodium benzoate, improved the recovery rate by several %, and the measurements with the reagents of Examples 6 to 9 containing 5.6 mM or more of sodium benzoate improved the recovery rate by 10% or more.

## Claims

1. A method for quantifying protein in a specimen using a complex in which a metal is coordinated with a dye and of which an absorption wavelength shifts when the protein binds to the complex, the method comprising reacting the protein with the complex in the presence of an aromatic carboxylic acid or a salt thereof.

2. A method for improving false high values when quantifying protein in a specimen using a complex in which a metal is coordinated with a dye and of which an absorption wavelength shifts when the protein binds to the complex, the method comprising reacting the protein with the complex in the presence of an aromatic carboxylic acid or a salt thereof.

3. The method according to claim 1 or 2, wherein the aromatic carboxylic acid or the salt thereof is a monocyclic or bicyclic aromatic carboxylic acid which has two or less carboxyl groups, and has no other substituents or an aromatic ring substituted with a sulfonic acid group, a hydroxyl group, a halogen, or an alkyl group having 1 to 4 carbon atoms.

4. The method according to claim 1 or 2, wherein the aromatic carboxylic acid or the salt thereof comprises one or more selected from benzoic acid, hydroxybenzoic acid, aminobenzoic acid, salicylic acid, phthalic acid, and salts thereof.

5. The method according to claim 4, wherein the aromatic carboxylic acid or the salt thereof comprises one or more selected from benzoic acid, hydroxybenzoic acid, salicylic acid, phthalic acid, and salts thereof.

6. The method according to any one of claims 1 to 5, wherein the aromatic carboxylic acid or the salt thereof is contained in a reaction solution at a concentration of 4.2 mM or more.

7. The method according to any one of claims 1 to 6, wherein the protein is reacted with the complex in the presence of
(1) an aliphatic amine having at least one substituent selected from an alkyl group having 1 to 4 carbon atoms, a hydroxylamino group having 1 to 4 carbon atoms and an aminoalkyl group having 1 to 4 carbon atoms, or a salt thereof (R-NH-R),
(2) aniline, an aniline derivative having at least one substituent selected from a sulfonic acid group, a hydroxyl group, a sulfonylamino group, a halogen, an alkyl group having 1 to 4 carbon atoms, a hydroxylamino group having 1 to 4 carbon atoms and an amino group, or a salt thereof (R-NH-substituted or unsubstituted phenyl group or phenylene group-R),
(3) hydrazine or a hydrazine derivative in which hydrazine is substituted with an alkyl group having 1 to 4 carbon atoms,
(4) thiourea or a thiourea derivative in which thiourea is substituted with an alkyl group having 1 to 4 carbon atoms,
(5) a polyhydric alcohol, and/or
(6) a chelating agent,
as well as the aromatic carboxylic acid or the salt thereof.

8. The method according to any one of claims 1 to 7, wherein the dye is pyrogallol red or pyrocatechol violet, and the metal is molybdenum, tin, or iron.

9. The method according to any one of claims 1 to 8, wherein the specimen is urine or spinal fluid.

10. A reagent for measuring protein, comprising: a complex in which a metal is coordinated with a dye and of which an absorption wavelength shifts when the protein binds to the complex; and an aromatic carboxylic acid or a salt thereof.

11. The reagent for measuring protein according to claim 10, wherein the aromatic carboxylic acid or the salt thereof is a monocyclic or bicyclic aromatic carboxylic acid which has two or less carboxyl groups, and has no other substituents or an aromatic ring substituted with a sulfonic acid group, a hydroxyl group, a halogen, or an alkyl group having 1 to 4 carbon atoms.

12. The reagent for measuring protein according to claim 10, wherein the aromatic carboxylic acid or the salt thereof comprises one or more selected from benzoic acid, hydroxybenzoic acid, aminobenzoic acid, salicylic acid, phthalic acid, and salts thereof.

13. The reagent for measuring protein according to claim 12, wherein the aromatic carboxylic acid or the salt thereof comprises one or more selected from benzoic acid, hydroxybenzoic acid, salicylic acid, phthalic acid, and salts thereof.

14. The reagent for measuring protein according to any one of claims 10 to 13, wherein the aromatic carboxylic acid or the salt thereof is contained at a concentration of 4.2 mM or more.

15. The reagent for measuring protein according to any one of claims 10 to 14, wherein the dye is pyrogallol red or pyrocatechol violet, and the metal is molybdenum, tin, or iron.

16. The reagent for measuring protein according to any one of claims 10 to 15, further comprising:
(1) an aliphatic amine having at least one substituent selected from an alkyl group having 1 to 4 carbon atoms, a hydroxylamino group having 1 to 4 carbon atoms and an aminoalkyl group having 1 to 4 carbon atoms (R-NH-R); or a salt thereof;
(2) aniline; an aniline derivative in which aniline is substituted with a hydroxyl group, or at least one substituent selected from a hydroxyl group, a sulfonylamino group, a halogen, an alkyl group having 1 to 4 carbon atoms, a hydroxylamino group having 1 to 4 carbon atoms and an amino group (R-NH-substituted or unsubstituted phenyl or phenylene residue-R); or a salt thereof;
(3) hydrazine, a hydrazine derivative in which hydrazine is substituted with an alkyl group having 1 to 4 carbon atoms (R-NH-NH₂), or a salt thereof;
(4) thiourea, a thiourea derivative in which thiourea is substituted with an alkyl group having 1 to 4 carbon atoms(R-NH-C(=S)-N(-R)-R), or a salt thereof;
(5) a polyhydric alcohol; and/or
(6) a chelating agent.

17. The reagent for measuring protein according to any one of claims 10 to 16, which is used for measuring protein in urine or a sample derived from urine.

18. A method for producing a reagent for measuring protein, the method comprising dissolving a dye selected from pyrocatechol violet, pyrogallol red, bromopyrogallol red, o-hydroxyhydroquinonephthalein, and gallein; an oxyacid salt, a halide, a complex salt or an organic or inorganic acid salt of a metal selected from molybdenum, tin or iron; and an aromatic carboxylic acid or a salt thereof in a buffer solution.
